# EUROPEAN PATENT SPECIFICATION

(11) **EP 3 082 685 B1**
(45) Date of publication and mention of the grant of the patent: **07.03.2018**
(21) Application number: 13899721.8
(22) Date of filing: 18.12.2013
(51) Int. Cl.: A61F 13/472, A61F 13/534, A61F 13/535

(54) **ABSORBENT ARTICLE HAVING A HUMP**
SAUGFÄHIGER ARTIKEL MIT EINEM HÖCKER
ARTICLE ABSORBANT POSSÉDANT UNE BOSSE

(43) Date of publication of application: 26.10.2016
(73) Proprietor: SCA Hygiene Products AB, 405 03 Göteborg (SE)
(72) Inventor: KNÖS, Anna, S-405 03 Göteborg (SE); PALMQVIST, Lisa, S-405 03 Göteborg (SE); HALLBERG, Patrik, S-405 03 Göteborg (SE); BAGGER-SJÖBÄCK, Anna, S-405 03 Göteborg (SE)
(74) Representative: Hoffmann Eitle
(86) International application number: PCT/SE2013/051557
(87) International publication number: WO 2015/094044

(56) References cited:
- EP-A1- 0 768 072
- EP-A2- 0 613 671
- WO-A1-01/24754
- WO-A1-2013/159295
- US-A1- 2003 125 701
- US-A1- 2004 140 048
- US-A1- 2004 186 449

## Description

### TECHNICAL FIELD

The present invention relates to an improved absorbent article comprising a hump.

### BACKGROUND

Absorbent articles for hygiene purposes are typically intended to absorb body liquids, such as urine and blood. Users put high demands on such articles, requiring them to be relatively thin and comfortable and at the same time to effectively absorb body fluids without any risk of leakage.

Absorbent articles, such as sanitary napkins, diapers, incontinence guards or the like typically include a liquid pervious topsheet, intended to be facing the wearer during use, a liquid impervious backsheet and an absorbent structure there between.

A common problem associated with absorbent articles of this type is that the articles undesired deforms during use since the article is pressed together between the user's legs. The articles may crumble up during use leading to formation of wrinkles on the top surface of the article and channels in the absorbent article in an uncontrolled manner. This in turn leads to that fluid may flow out on the articles side edges and create leakage.

Absorbent structures commonly used have different shapes and constitute of different material, and may comprise suberabsorbents. For improving the fit of the articles different solutions have been provided. For example patent application WO03/053301 describes absorbent articles which comprise a stiffening element that is intended to contribute to the three-dimensional shape of the articles during their use.

US 2004/140048 A1 and US 2003/125701 A1 are further prior art.

Nevertheless, todays absorbent articles still have the problem of crumbling up during use, due to the squeezing forces in the lateral direction in the crotch portion of the article, generated by the thighs of the wearer. A crumbled up article is uncomfortable for the wearer and have a reduced absorption capacity and an increased risk of leakage. Furthermore, the absorbent structure in many cases has a too low absorption speed in order to be able to absorb the large amount of fluid that can be discharged without causing leakage.

One way of enhancing the absorption speed of the article is to incorporate a hump element between the topsheet and backsheet of the absorbent article. However, the hump elements according to prior art often creates material waste during the manufacturing process, as the rest of the material cannot be used further after the hump structure has been cut from a material base sheet. Furthermore, the problem of leakage is still occurring with these prior art hump elements and there is therefore still a need for an improved hump element for hygiene absorbent articles, in order to prevent leakage of the articles and in order to enable a manufacturing method with reduced waist material.

### SUMMARY

In view of the above-it is desired to provide an improved hump for an absorbent article.

This is achieved by an absorbent article according to appended claim 1. Embodiments are set forth in the appended dependent claims and in the following description and drawings.

An aspect relates to an absorbent article, the absorbent article being a personal hygiene article, which article has a longitudinal direction and a transverse direction, a front portion, a rear portion and a crotch portion located between the rear portion and the front portion. The article comprising a liquid permeable topsheet, a liquid impermeable backsheet, and an absorbent core enclosed between the topsheet and the backsheet, and a hump arranged substantially in the crotch portion between the top sheet and the backsheet and extending in the longitudinal direction of the article. The hump has an essentially rectangular form with a first and a second longitudinal side edge extending in the longitudinal direction of the article, a front transverse side edge extends in the transverse direction towards the front portion of the article, a back transverse side edge extending in the transverse direction towards the rear portion of the article. The front transverse side edge of said hump comprises a protuberance and said back transverse side edge of said hump comprises a recess.

The absorbent article is an article that is intended to absorb and contain exudates discharged from the body of a wearer. The article is intended for being worn inside garments such as ordinary underpants. The article may be disposable, i.e. intended to be discarded after a single use and not intended to be laundered or otherwise restored or reused.

A hump is in this context meant to be an element having a three-dimensional structure, i.e. having an extension in the z-direction.

According to one embodiment the hump may be arranged between the topsheet and the absorbent core.

The hump according to the invention, being positioned in the crotch portion of the absorbent article, prevents the absorbent article from crumbling up during use. This is due to the form and design of the hump having a front transverse side edge comprising a protuberance and a back transverse side edge of the hump comprising a recess. The design of the hump prevents the absorbent article from being squeezed by the squeezing forces in the lateral direction in the crotch portion of the article, generated by the thighs of the wearer. The form of the hump creates a raised portion within the absorbent article which together with the non-squeezing effect results in an improved position of the absorbent article to the wearer's genitals.

The absorbent article is prevented from crumbling up and this leads to no reduction of the receiving area for the fluid. Accordingly, the absorbent article will have a substantially even and large top surface in a substantially horizontal plane. In this context, the receiving area means the surface of the absorbent article which is facing a wearer during use, and which surface is extending in a substantially horizontal plane and hence will receive the extruded fluid from the wearer.

A horizontal plane is by definition a plane extending in the longitudinal and the transverse direction of the article.

An advantage of a large receiving area of the absorbent article, is that the capability of the absorbent article to quickly receive liquid, distribute the liquid and drain the fluid into the core will be high. This in turn will enhance the absorption speed and reduce the risk of leakage. Furthermore, due to the recess in the back transverse side edge of the hump, also the rear portion of the absorbent article will stay close to the body during use and adapt an improved fit between the buttocks of the wearer, and hence reduce the risk of leakage. During use of an absorbent article, a fold in the rear portion of the article is desired. The recess in the back transverse side edge of the hump enables the hump to extend in a longitudinal rear direction of the absorbent article further back and in connection with the fold that penetrates the cleft between the buttocks of the wearer. That means that the receiving area of the article is further enhanced in a transversal and a longitudinal direction of the article. Accordingly, when fluid is discharged from a wearer, the fluid is prevented from flowing through the cleft of the wearer to the articles longitudinal side edge, but will instead be captured by the hump. Accordingly, the recess in the back transverse side edge of the hump results in an article that curves and shapes itself in a desired manner, when fitted on the wearer.

According to one embodiment of the invention the protuberance may have a convex shape.

In this context, a convex shape is an outline that is curved or rounded outward relative to the hump. Outward means in this context, rounded in the front portion direction of the absorbent article in a horizontal plane.

According to a further embodiment of the invention said recess may have a concave shape.

In this context, the definition of a concave shape is an outline that is curving or rounded inward relative to the hump. A concave form is the opposite of a convex form, as defined above. Inward means in this context, the curving is extending in the front portion direction of the absorbent article, defined in a horizontal plane.

Further, when a wearer is going to place the absorbent article inside the brief, the shape of the hump indicate the front portion and the back portion of the absorbent article. Accordingly, the absorbent article enables the wearer to understand in what direction to place the article inside the brief. Furthermore, the design of the hump enables the wearer to visually understand that the hump is going to be placed in the wetting point. Therefore, a reduced risk of an incorrect position of the article is achieved.

In this context, a wetting point is the area of the article within which fluid mainly can be expected to be discharged when the absorbent article is correctly positioned on the wearer.

Furthermore, the article having a horizontal plane extending in said longitudinal and transverse direction of the article, wherein said protuberance extending substantially in said horizontal plane.

According to an embodiment of the invention the article may have a horizontal plane extending in said longitudinal and transverse direction of the article, wherein said recess extending substantially in said horizontal plane.

According to a further embodiment of the invention said protuberance may comprise a contour extending between said first and second longitudinal side edge of the hump, said recess comprising a contour extending between said first and second longitudinal side edge of the hump, wherein the respective contours are essentially identical. The respective contours may be essentially identical which lead to a simplified and cost saving manufacturing method. This is accomplished by delimiting the method step of a transverse cutting in order to form a single hump. When a transverse cutting step in the hump manufacturing method is provided, both the front transverse side edge of a first hump and the back transverse side edge of a second hump may be provided in a single cutting step. Accordingly, a minimum of waste material and huge costs and environmental benefits are achieved.

According to one embodiment of the invention said protuberance comprising a contour extending between said first and second longitudinal side edges of the hump, said contour may have a sine shape.

A sine shape is by definition a sinusoidal shaped wave.

According to a further embodiment of the invention said recess comprising a contour extending between said first and second longitudinal side edge of the hump, said contour has a sine shape.

According to an embodiment of the invention said absorbent article further comprises an additional layer positioned between said liquid permeable top sheet and said hump.

According to a further embodiment of the invention the absorbent article comprises an additional layer positioned between said hump and said absorbent core.

The additional layer may be a liquid acquisition layer.

According to an embodiment of the invention a distance A between said front transverse side edge of said hump and said back transverse side edge of said hump may be between 20 mm and 120 mm, preferably between 40 mm and 100 mm and more preferably between 60 mm and 80 mm.

According to a further embodiment of the invention said hump may further comprise a first point of intersection between said first longitudinal side edge of the hump and said back transverse side edge of the hump, a second point of intersection between said second longitudinal side edge of the hump and said back transverse side edge of the hump, and an imaginary straight line L1 extending in the transverse direction of said article from said first point of intersection to said second point of intersection and an imaginary point Z1 positioned in the centre of said imaginary straight line L1. The front transverse side edge of the hump may further comprise an outermost point positioned in a longitudinal direction nearest the front portion of the article. The distance A may be the longest distance of the hump in a longitudinal direction of the article between said outermost point and said imaginary point Z1.

According to another embodiment of the invention the hump may be a thickness B between 0,5 mm and 30 mm, preferably between 1 mm and 10 mm and more preferably between 2 mm and 5 mm.

The three-dimensional form and design of the hump creates a free volume within the absorbent article and therefore enhance the liquid dispersion capacity of the article in the z-direction. This in turn provides that the absorbent article has sufficient admission capacity or temporary retention capacity. Accordingly, an absorbent article with a reduced risk of leakage is achieved.

According to a further embodiment of the invention a distance C between said first and second longitudinal side edge of said hump may be between 20 mm and 70 mm, preferably between 30 mm and 40 mm.

Furthermore the distance may be the longest distance in a transverse direction of the hump between said first and second longitudinal side edge of said hump.

According to another embodiment of the invention the back transverse side edge may further comprise an innermost point Y1 positioned in a longitudinal direction nearest the front portion of the article. Furthermore a distance between said innermost point Y1 and said imaginary point Z1 may be between 5 mm and 50 mm and more preferably between 10 mm and 25 mm.

According to a further embodiment of the invention the hump may further comprise a third point of intersection between said first longitudinal side edge of the hump and said front transverse side edge of the hump, a fourth point of intersection between said second longitudinal side edge of the hump and said front transverse side edge of the hump, an imaginary straight line L2 extending in the transverse direction of said article from said third point of intersection to said fourth point of intersection, and an imaginary point Z2 positioned in the centre of said imaginary straight line L2. A distance D2 between said outermost point Y2 and said imaginary point Z2 may be between 5 mm and 50 mm and more preferably between 10 mm and 25 mm.

According to another embodiment of the invention the distance D1 may be identical to said distance D2.

Advantageously, this embodiment allows the total length of the hump being longer without corresponding increase in material consumption.

According to another embodiment of the invention the hump may be of a nonwoven high loft material, an air laid material or a foam material.

### BRIEF DESCRIPTION OF THE DRAWINGS

The present invention will now be described in more detail with reference to the accompanying schematic drawings which show preferred embodiments of the invention:
Figure 1A shows schematically a perspective view of a prior art absorbent article during use;
Figure 1B shows schematically a perspective view of an absorbent article according to an embodiment of the the present invention during use;
Figure 2 shows schematically a perspective view of an embodiment of an absorbent article according to the present invention;
Figure 3 shows a plan view of an absorbent article according to an embodiment of the present invention;
Figure 4 shows a plan view of a hump according to an embodiment of the present invention;
Figure 5A-C shows schematically a cross-sectional view of the absorbent article of Figure 2, along the line III-III, and
Figure 6 illustrate schematically one method by which hump elements according to the present invention may be manufactured.

### DETAILED DESCRIPTION OF THE INVENTION

The absorbent article of the invention being a personal hygiene article may be any type of incontinence protectors, sanitary napkins, panty liners or a diaper. Preferably the absorbent article may be a sanitary napkin.

The absorbent article comprises a liquid permeable top sheet, a liquid impermeable backsheet, and an absorbent core enclosed between the top sheet and the backsheet, and a hump arranged between the top sheet and the backsheet. The hump may be of a nonwoven high loft material, an airlaid material or a foam material or other suitable material having the similar properties. The foam material may for example be a polyurethane foam.

The topsheet and the backsheet of the absorbent article may extend together laterally outside of the absorbent core along the whole circumference of the absorbent core and be connected to each other in an edge joint around the periphery of the absorbent core. The backsheet may preferably cover part of the topsheet to form an edge barrier. The edge joint may be formed in any suitable manner as known in the art such as by means of adhesive, ultrasonic bonding, thermo-bonding, stitching, etc. Alternative covering arrangements such as wrapped-around covers are also conceivable within the scope of the invention. Furthermore, alternatively, a liquid acquisition layer may be positioned between the topsheet and the backsheet and may extend to the two longitudinal side edges of the absorbent article.

The topsheet may consist of any material which is suitable for the purpose, i.e. be soft and liquid pervious. Examples of commonly found topsheet materials are nonwoven materials, perforated plastic films, plastic or textile mesh, and fluid permeable foam layers. Laminates consisting of two or more topsheet materials are also commonly employed, as are topsheets consisting of different materials within different parts of the fluid permeable wearer-facing surface.

The backsheet is fluid impermeable. However, backsheet materials that are only fluid repellant may be used particularly in instances where relatively small amounts of fluid are expected to be taken up. The backsheet is commonly constituted by a thin, flexible, fluid-impermeable plastic film, but fluid-impermeable nonwoven materials, fluid impermeable foams and fluid impermeable laminates are also contemplated within the scope of the invention. The backsheet may be breathable, implying that air and vapor may pass through the backsheet. Alternatively, the backsheet may not be breathable. Furthermore, the backsheet may have an outer, garment-facing surface of a textile material such as nonwoven.

The absorbent core may be made up of any suitable absorbent or fluid uptake material as known in the art, such as one or more layers of cellulose fluff pulp, foam, fiber waddings, etc. The absorbent core may contain fibers or particles of highly absorbent polymer material, commonly known as superabsorbents, which are materials having the ability to absorb and retain large quantities of fluid upon formation of a hydrogel. The superabsorbents may be mixed with cellulose fluff pulp and/or may be arranged in pockets or layers in the absorbent core. The fibres may be pulp fibres and the superabsorbent material may be polyacrylate-based particles.

The absorbent core may further incorporate components for improving the properties of the absorbent core. Some examples of such components are binder fibers, fluid-dispersing materials, wetness indicators, fluid acquisition materials, etc., as known in the art.

The absorbent core may also be a stiffening element and be designed so as to be stiff in order as far as possible to avoid the absorbent article being compressed in an uncontrolled manner. The shape of the absorbent core may be an oval, rectangle or another geometrical design adapted to improve fit. An example of an absorbent article having a stiffening element is described in WO 2008/004961.

The absorbent article typically, in case of a pad or a sanitary napkin, has an elongate, generally rectangular shape when fully extended in all directions. In this context, a generally rectangular shape is intended to encompass also that, for instance, the corners of the absorbent article may be rounded, or that the edges of the absorbent article may not be completely linear. Accordingly, any suitable shape may be used for the absorbent article, such as hourglass shape, trapezoidal shape, triangular shape an oval shape, etc., The shape of the article of the invention may be symmetrical about a transverse center line through the article, or may be asymmetrical with end portions having differing shapes and/or differing sizes.

The absorbent article may have two longitudinal side edges having equal length and extending generally in the same direction as a longitudinal center line through the absorbent article. Front and rear end edges typically extend transversely to the longitudinal center line at the ends of the absorbent article. The rear end edge is intended to be orientated rearwards during use of the absorbent article, and the front end edge is intended to be facing forwards towards the abdomen of the wearer.

The absorbent article may have a front end portion, a rear end portion and a crotch portion located intermediate the end portions, the crotch portion being a portion, which is intended to be placed against the crotch of a wearer during use of the article and to constitute the main acquisition area for body fluid that reaches the absorbent article.

The absorbent article may further include fastening means for fastening of the absorbent article inside a supporting pant garment, such as a pair of underpants. The fastening means may be in the form of two longitudinally extending bands of pressure sensitive adhesive arranged on the garment-facing surface of the backsheet. The fastening means can be covered by a releasable protective layer, e.g. a siliconized paper, a nonwoven or any other releasable material as is known in the art. Before placing the absorbent article in the supporting pant garment, the protective layer is removed from the fastening means to expose the adhesive and make it available for fastening to the pant garment.

The fastening means is optional to the invention and may be omitted, if desired. When using an adhesive fastening means, any suitable adhesive pattern may be used such as full coating of the backsheet, one or more longitudinal adhesive band, transverse bands, dots, circles, curves, stars, etc. Furthermore, the fastening means may be a mechanical fastener such as hook-type fasteners, clips, press studs, etc. or may be a frictional fastener such as a frictional coating or an open-celled foam. Combinations of different types of fasteners are also conceivable.

The hump is sandwiched between the top sheet and the backsheet of the absorbent article and may be located above the absorbent core. Alternatively, the hump may be located under the absorbent core. The absorbent article may comprise an additional layer positioned between said top sheet and the hump. Alternatively the additional layer could be positioned between the hump and the absorbent core. The absorbent article may also not comprise an additional layer. The additional layer could be a liquid acquisition layer and may comprise a single layer or multiple layers, such as an upper acquisition layer facing towards the wearer's skin and a lower acquisition layer facing the garment of the wearer. The liquid acquisition layer may consist of, for example, a nonwoven high loft material, an airlaid material or a foam material or other suitable material having the similar properties. The airlaid material may be an airlaid fibrous material of low density bonded together with bonding agent and/ or thermofibre. The hump may be of the same material as the liquid acquisition layer. Alternatively, the liquid acquisition layer and the hump may be of different materials. The absorbent core may have a higher capillary force than the liquid acquisition layer and the hump, located above and therefore draws liquid from the liquid acquisition layer and the hump and prevents back-wetting by liquid from the absorbent core to the liquid acquisition layer or the hump and to the topsheet which remains essentially dry during use of the product. Only when the absorbent element is saturated with liquid can transport take place from the absorbent core to the liquid acquisition layer and / or the hump.

The hump is arranged substantially in the crotch portion of the absorbent article. The hump may extend into the front portion of the absorbent article. The hump may extend into the back portion of the absorbent article. Alternatively the hump may extend in both the front portion and the back portion of the absorbent article at the same time.

The invention will now be described by means of example referring to the accompanying figures. In this example of the invention the absorbent article 1 is a sanitary napkin 1.

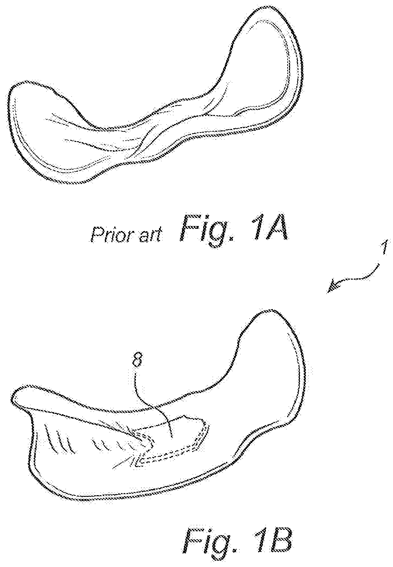

Figure 1A illustrates schematically a perspective view of a prior art absorbent article during use. The wearer of the absorbent article is not included in the figure in order to in a clearer manner, illustrate how the absorbent article is formed and crumbled up during use. The result is an article with an uncontrolled crease which may cause uncontrolled liquid flow on the surface of the article.

Figure 1B illustrates schematically a perspective view of an embodiment of an absorbent 1 article according to the invention, during use. The wearer of the absorbent article is not included in the figure in order to in a clearer manner, illustrate how the absorbent article is formed during use. The hump 8 is arranged in the crotch portion 3 of the article 1 between the topsheet 5 and the backsheet 6. The article 1 curves and shapes in a desired manner and a fold in the rear portion of the article is formed in desired manner.

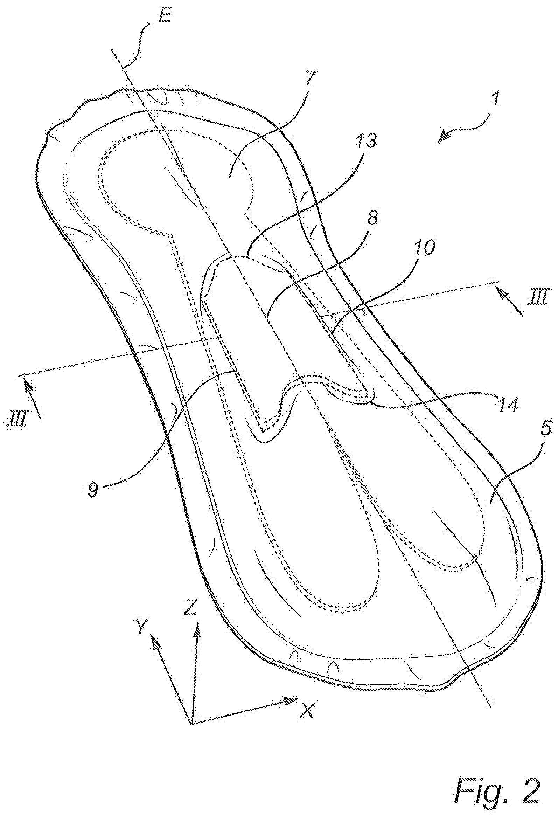

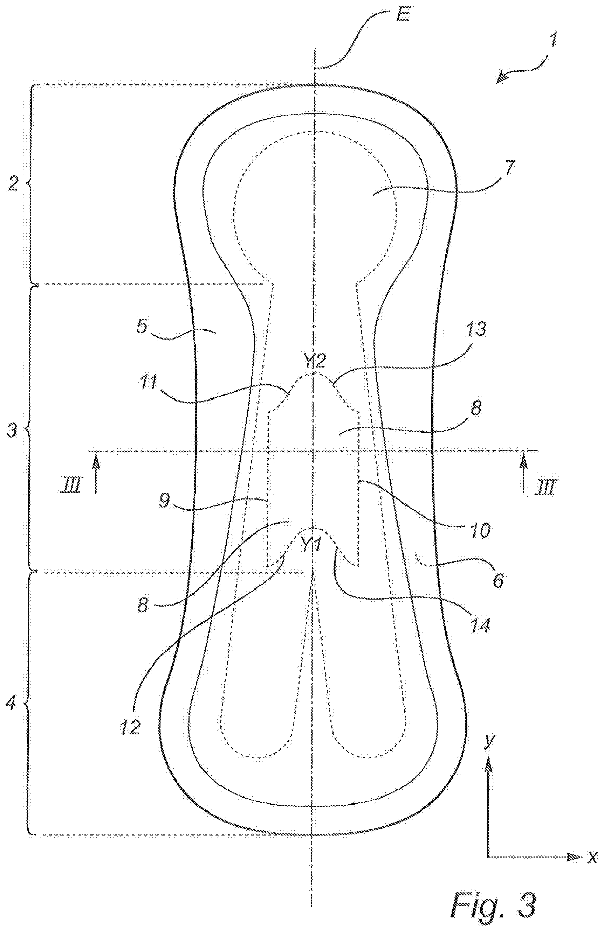

Figure 2 shows a perspective view of an embodiment of the absorbent article 1 according to the present invention and figure 3 shows a plan view of an absorbent article 1 according to an embodiment of the present invention, both figures 2 and 3 are seen from the surface of the absorbent article that is intended to be facing towards a wearer's body when being worn. The article 1 has a transverse direction X, a longitudinal direction Y and a longitudinal centre line E as indicated. The article 1 further has a front portion 2, a crotch portion 3 and a rear portion 4 arranged in the article's longitudinal direction. In use, the front portion 2 of the article 1 is intended to cover the pubic region of the wearer. The crotch portion 3 of the article 1 is located adjacent to the front portion 2 in the longitudinal direction Y. In use, the crotch portion 3 lies between the legs of the wearer and cover the wearers genital region. The rear portion 4 is located at the opposite end of the article 1 from the front portion 2 and is located adjacent to the crotch portion 3 in the longitudinal direction Y. In use the rear portion 4 extends towards the user's rear. Furthermore the absorbent article 1 comprises a fluid permeable topsheet 5, disposed at the top surface of the absorbent article 1 which is intended to be facing a wearer of the absorbent article 1, a fluid impermeable backsheet 6 disposed at the lower back side of the absorbent article 1 that is intended to be facing the undergarment of the wearer, and an absorbent core 7, enclosed between the topsheet 5 and the backsheet 6. Between the topsheet 5 and the backsheet 6 a hump 8 is arrange and according to this embodiment the hump 8 is situated above the core 7. The hump 8 has a first longitudinal side edge 9 and a second longitudinal side edge 10 extending in the longitudinal direction Y of the article 1. The hump 8 has a front transverse side edge 11 extending in the transverse direction X towards the front portion 2 of the article 1. Towards the front portion 2 is meant to be in a direction towards the front portion 2 of the article 1 but not necessary all the way into the front portion 2 of the article 1. The front transverse side edge 11 comprises a protuberance 13 extending in a horizontal plane and having a convex shape. The hump further has a back transverse side edge 12 extending in the transverse direction X towards the rear portion 4 of the article 1. Towards the rear portion 4 is meant to be in a direction towards the rear portion 4 of the article 1 but not necessary all the way into the rear portion 4 of the article 1. The back transverse side edge 12 comprises a recess 14 extending in a horizontal plane and having a concave shape.

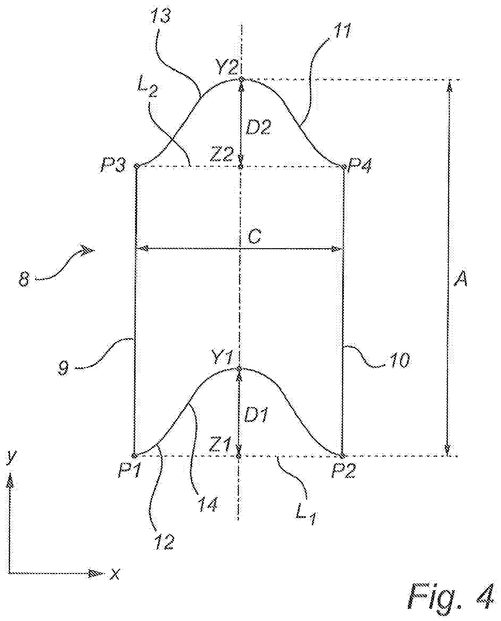

Figure 4 shows a plan view of a hump 8 according to an embodiment of the present invention. 4. The hump 8 in figure 4 is an enlargement of the hump 8 in figure 2, even though the absorbent article 1 is not shown in figure 4. The hump 8 has a first longitudinal side edge 9 and a second longitudinal side edge 10 extending in the longitudinal direction Y of the article 1. The hump 8 has a front transverse side edge 11 extending in the transverse direction X in the front portion 2 of the article 1. The front transverse side edge 11 comprises a protuberance 13 extending in a horizontal plane and having a convex shape. The hump 8 further has a back transverse side edge 12 extending in the transverse direction X in the rear portion 4 of the article 1. The back transverse side edge 12 comprises a recess 14 extending in a horizontal plane and having a concave shape. The recess 14 comprises a contour which extends between the first longitudinal side edge 9 and the second longitudinal side edge 10 and the contour has a shine shape. The protuberance 13 comprises a contour which extends between the first longitudinal side edge 9 and the second longitudinal side edge 10 and the contour has a shine shape. The hump 8 has a first point of intersection P1 between the first longitudinal side edge 9 and the back transverse side edge 12 and a second point of intersection P2 between the second longitudinal side edge 10 and the back transverse side edge 12. An imaginary straight line L1 extends in the transverse direction X of the article 1 from the first point of intersection P1 to the second point of intersection P2. An imaginary point Z1 is positioned in the centre of the imaginary straight line L1. The imaginary point Z1 divides the imaginary straight line L1 in two equally long line parts. The front transverse side edge 11 comprises an outermost point Y2 intended to be positioned in the longitudinal direction Y nearest the front portion 2 of the article 1, when the hump 8 is arranged in the absorbent article 1 (see figure 3). The distance A of the hump 8 is the longest distance of the hump in the longitudinal direction Y, between the outermost point Y2 and the imaginary point Z1. The back transverse side edge 12 comprises an innermost point Y1 intended to be positioned in the longitudinal direction Y nearest the front portion 2 of the article 1, when the hump 8 is arranged in the absorbent article 1 (see figure 3). A distance D1 between the innermost point Y1 and the imaginary point Z1 is between 5 mm and 50 mm and preferably between 10 mm and 25 mm. The hump 8 further comprises a third point of intersection P3 between the first longitudinal side edge 9 and the front transverse side edge 11 and a fourth point of intersection P4 between the second longitudinal side edge 10 of the hump 8 and the front transverse side edge 11 of the hump 8. An imaginary straight line L2 extends in the transverse direction X from said third point of intersection P3 to the fourth point of intersection P4. An imaginary point Z2 is positioned in the centre of the imaginary straight line L2. The imaginary point Z2 divides the imaginary straight line L2 in two equally long line parts A distance D2 between the outermost point Y2 and the imaginary point Z2 is between 5mm and 50 mm and preferably between 10 mm and 25 mm. Distance D1 is in the embodiment according to figure 4 identical with distance D2. The distance C is the transversal X distance between the first and second longitudinal side edges 9, 10 of the hump 8, i.e. C is the width of the hump 8.

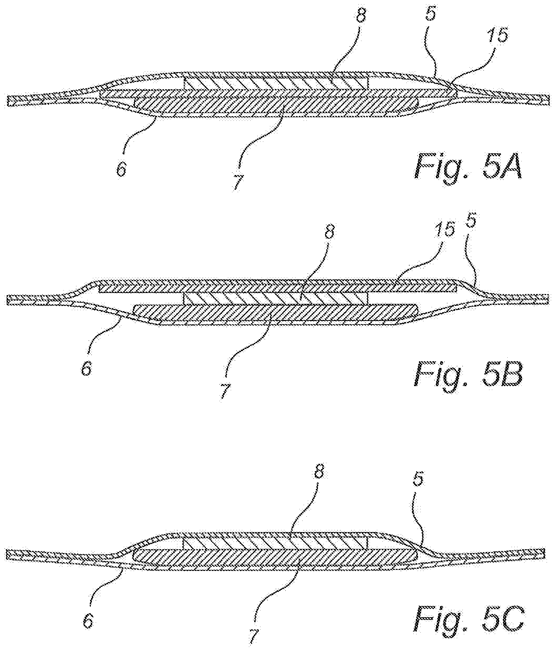

Figure 5A-C is a transverse cross-sectional view of an absorbent article 1, such as the sanitary napkin 1 shown in figure 3, through the mid-point of the article 1. It illustrates a liquid-permeable topsheet 5, a backsheet 6 and an absorbent core 7 enclosed between the liquid-permeable topsheet 5 and the backsheet 6. In the embodiment illustrated in Figure 5A, a liquid acquisition layer 15 is arranged on top of the absorbent core 7 and the hump 8 is arranged above the liquid acquisition layer 15. In the embodiment illustrated in Figure 5B, a liquid acquisition layer 15 is arranged on top of the hump 8 and the hump 8 is arranged on the absorbent core 7. Hence the hump 8 is sandwich between the liquid acquisition layer 15 and the core 7. In the embodiment illustrated in Figure 5C, a hump 8 is arranged on top of the absorbent core 7. Hence the hump 8 is sandwich between topsheet 5 and the core 7.

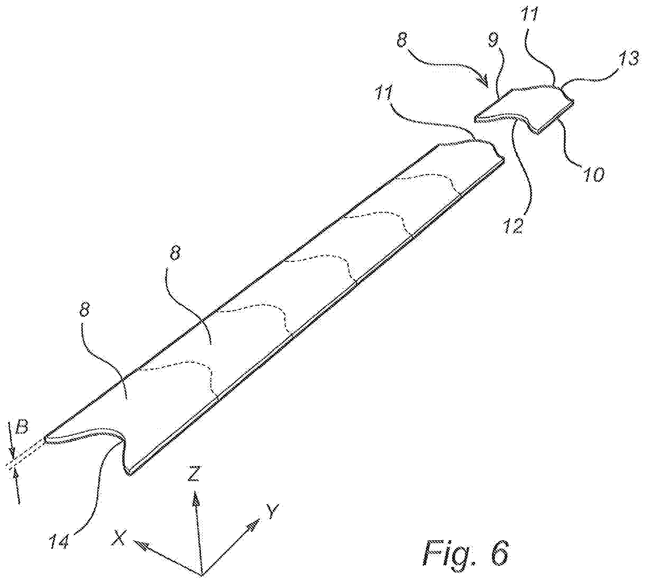

Figure 6 illustrate schematically one method by which hump elements 8 according to the present invention may be manufactured. The humps 8 have a first longitudinal side edge 9 and a second longitudinal side edge 10 extending in the longitudinal direction Y of the article 1. The humps 8 have a front transverse side edge 11 extending in the transverse direction X towards the front portion 2 of the article 1 and a back transverse side edge 12 extending in the transverse direction X towards the rear portion 4 of the article 1. The front transverse side edge 11 comprises a protuberance 13 extending in a horizontal plane and having a convex shape. The back transverse side edge 12 comprises a recess 14 extending in a horizontal plane and having a concave shape. The protuberance 13 comprises a contour which extends between the first and second longitudinal side edge 9, 10 and the recess comprises a contour which extends between the first and second longitudinal side edge 9, 10. The respectively contours are essentially identical which leads to that the method step of a transverse cutting in order to form a single hump is simplified. Both the front transverse side edge 11 of a first hump 8 and the back transverse side edge 12 of a second hump 8 may be provided in a single cutting. The distance B is the thickness of the hump 8, i.e. the extension in the z-direction as indicated in the figure.

## Claims

1. An absorbent article (1), the absorbent article (1) being a personal hygiene article, which article has a longitudinal direction (Y) and a transverse direction (X), a front portion (2), a rear portion (4) and a crotch (3) portion located between the rear portion (4) and the front portion (2), the article comprising a liquid permeable topsheet (5), a liquid impermeable backsheet (6), and an absorbent core (7) enclosed between the topsheet (5) and the backsheet (6), and a hump (8) arranged substantially in the crotch portion (3) between the topsheet (5) and the backsheet (6) and extending in the longitudinal direction (Y) of the article
**characterised by**
said hump (8) having an essentially rectangular form with a first and a second longitudinal side edge (9,10) extending in the longitudinal direction (Y) of the article (1), a front transverse side edge (11) extending in the transverse direction (X) towards the front portion (2) of the article (1), a back transverse side edge (12) extending in the transverse direction (X) towards the rear portion (4) of the article (1), wherein said front transverse side edge (11) of said hump (8) comprises a protuberance (13), wherein said protuberance (13) comprises a contour extending between said first and second longitudinal side edges (9, 10) of the hump (8), said contour has a sine shape, and said back transverse side edge (12) of said hump (8) comprises a recess (14), wherein said recess (14) comprises a contour extending between said first and second longitudinal side edge (9, 10) of the hump (8), said contour has a sine shape, wherein the respective contours are essentially identical,
said article (1) having a horizontal plane extending in said longitudinal and transverse direction (9, 10) of the article (1), wherein said protuberance (13) extends substantially in said horizontal plane; and/or
wherein said recess (14) extends substantially in said horizontal plane.

2. The absorbent article (1) of claim 1, wherein said hump (8) is arranged between the topsheet (5) and the absorbent core (7).

3. The absorbent article (1) according to any one of the preceding claims,
wherein said protuberance (13) has a convex shape.

4. The absorbent article (1) according to any one of the preceding claims,
wherein said recess (14) has a concave shape.

5. The absorbent article (1) according to any one of the preceding claims,
wherein said absorbent article (1) further comprising an additional layer (15) positioned between said liquid permeable topsheet (5) and said hump (8).

6. The absorbent article (1) according to any one of the preceding claims,
wherein said absorbent article (1) further comprising an additional layer (15) positioned between said hump (8) and said absorbent core (7).

7. The absorbent article (1) according to any of claim 5 or 6, wherein said additional layer (15) is a liquid acquisition layer (15).

8. The absorbent article (1) according to any one of the preceding claims,
wherein a distance (A) between said front transverse side edge (11) of said hump (8) and said back transverse side edge (12) of said hump (8) is between 20 mm and 120 mm, preferably between 40 mm and 100 mm and more preferably between 60 mm and 80 mm.

9. The absorbent article (1) according to claim 8, wherein said hump (8) further comprising a first point of intersection (P1) between said first longitudinal side edge (9) of the hump (8) and said back transverse side edge (12) of the hump (8), a second point of intersection (P2) between said second longitudinal side edge (10) of the hump (8) and said back transverse side edge (12) of the hump (8), and an imaginary straight line (L1) extending in the transverse direction (X) of said article (1) from said first point of intersection (P1) to said second point of intersection (P2)) and an imaginary point (Z1) positioned in the centre of said imaginary straight line (L1), wherein said front transverse side edge (11) of the hump (8) further comprising an outermost point (Y2) positioned in a longitudinal direction (Y) nearest the front portion (2) of the article (1), wherein said distance (A) is the longest distance of the hump (8) in a longitudinal direction (Y) of the article between said outermost point (Y2) and said imaginary point (Z1).

10. The absorbent article (1) according to any one of the preceding claims,
wherein said hump (8) has a thickness (B) between 0,5 mm and 30 mm, preferably between 1 mm and 10 mm and more preferably between 2 mm and 5 mm.

11. The absorbent article (1) according to any one of the preceding claims,
wherein a distance (C) between said first and second longitudinal side edge (9, 10) of said hump (8) is between 20 mm and 70 mm, preferably between 30 mm and 40 mm.

12. The absorbent article (1) according to claim 9, wherein said distance (C) is the longest distance in a transverse direction (X) of the hump (8) between said first and second longitudinal side edge (9, 10) of said hump (8).

13. The absorbent article (1) according to any one of the preceding claims,
wherein said back transverse side edge (12) of the hump (8) further comprising an innermost point (Y1) positioned in a longitudinal direction (Y) nearest the front portion (2) of the article (1), wherein a distance D1 between said innermost point (Y1) and said imaginary point (Z1) is between 5 mm and 50 mm and more preferably between 10 mm and 25 mm.

14. The absorbent article (1) according to any one of the preceding claims,
wherein said hump (8) further comprising a third point of intersection (P3) between said first longitudinal side edge (9) of the hump (8) and said front transverse side edge (11) of the hump (8), a fourth point of intersection (P4) between said second longitudinal side edge (10) of the hump (8) and said front transverse side edge (11) of the hump (8), an imaginary straight line (L2) extending in the transverse direction (X) of said article (1) from said third point of intersection (P3) to said fourth point of intersection (P4), and an imaginary point (Z2) positioned in the centre of said imaginary straight line (L2), wherein a distance D2 between said outermost point (Y2) and said imaginary point (Z2) is between 5 mm and 50 mm and more preferably between 10 mm and 25 mm.

15. The absorbent article (1) according to any of claims 13 and 14, wherein said distance D1 is identical with said distance D2.

16. The absorbent article (1) according to any one of the preceding claims,
wherein said hump (8) constitutes of a nonwoven high loft material, an airlaid material or a foam material.

## Patentansprüche

1. Saugfähiger Artikel (1), wobei der saugfähige Artikel (1) ein Körperhygieneartikel ist, wobei der Artikel eine Längsrichtung (Y) und eine Querrichtung (X), einen vorderen Abschnitt (2), einen hinteren Abschnitt (4) und einen Schrittabschnitt (3), der sich zwischen dem hinteren Abschnitt (4) und dem vorderen Abschnitt (2) befindet, aufweist, wobei der Artikel eine flüssigkeitsdurchlässige Decklage (5), eine flüssigkeitsundurchlässige Stützlage (6) und einen saugfähigen Kern (7), eingeschlossen zwischen der Decklage (5) und der Stützlage (6), und einen Höcker (8), im Wesentlichen in dem Schrittabschnitt (3) zwischen der Decklage (5) und der Stützlage (6) angeordnet und sich in die Längsrichtung (Y) des Artikels erstreckend, umfasst,
**dadurch gekennzeichnet, dass**
der Höcker (8) eine im Wesentlichen rechteckige Form, mit einem ersten und einem zweiten Längsseitenrand (9,10), die sich in die Längsrichtung (Y) des Artikels (1) erstrecken, einen vorderen quer verlaufenden Seitenrand (11), der sich in die Querrichtung (X) zu dem vorderen Abschnitt (2) des Artikels (1) erstreckt, einen rückseitigen quer verlaufenden Seitenrand (12), der sich in die Querrichtung (X) zu dem hinteren Abschnitt (4) des Artikels (1) erstreckt, aufweist, wobei der vordere quer verlaufende Seitenrand (11) des Höckers (8) eine Ausstülpung (13) umfasst, wobei die Ausstülpung (13) eine Kontur umfasst, die sich zwischen den ersten und zweiten Längsseitenrändern (9, 10) des Höckers (8) erstreckt, wobei die Kontur eine Sinusform aufweist, und der rückseitige quer verlaufende Seitenrand (12) des Höckers (8) eine Aussparung (14) umfasst, wobei die Aussparung (14) eine Kontur umfasst, die sich zwischen den ersten und zweiten Längsseitenrändern (9, 10) des Höckers (8) erstreckt, wobei die Kontur eine Sinusform aufweist, wobei die jeweiligen Konturen im Wesentlichen identisch sind,
wobei der Artikel (1) eine horizontale Ebene aufweist, die sich in die Längs- und Querrichtung (9, 10) des Artikels (1) erstreckt, wobei die Ausstülpung (13) sich im Wesentlichen in der horizontalen Ebene erstreckt; und/oder
wobei die Aussparung (14) sich im Wesentlichen in der horizontalen Ebene erstreckt.

2. Saugfähiger Artikel (1) nach Anspruch 1, wobei der Höcker (8) zwischen der Decklage (5) und dem saugfähigen Kern (7) angeordnet ist.

3. Saugfähiger Artikel (1) nach einem der vorstehenden Ansprüche, wobei die Ausstülpung (13) eine konvexe Form aufweist.

4. Saugfähiger Artikel (1) nach einem der vorstehenden Ansprüche, wobei die Aussparung (14) eine konkave Form aufweist.

5. Saugfähiger Artikel (1) nach einem der vorstehenden Ansprüche, wobei der saugfähige Artikel (1) weiter eine zusätzliche Lage (15) umfasst, die zwischen der flüssigkeitsdurchlässigen Decklage (5) und dem Höcker (8) positioniert ist.

6. Saugfähiger Artikel (1) nach einem der vorstehenden Ansprüche, wobei der saugfähige Artikel (1) weiter eine zusätzliche Lage (15) umfasst, die zwischen dem Höcker (8) und dem saugfähigen Kern (7) positioniert ist.

7. Saugfähiger Artikel (1) nach einem von Anspruch 5 oder 6, wobei die zusätzliche Lage (15) eine Flüssigkeitsaufnahmelage (15) ist.

8. Saugfähiger Artikel (1) nach einem der vorstehenden Ansprüche, wobei ein Abstand (A) zwischen dem vorderen quer verlaufenden Seitenrand (11) des Höckers (8) und dem rückseitigen quer verlaufenden Seitenrand (12) des Höckers (8) zwischen 20 mm und 120 mm, vorzugsweise zwischen 40 mm und 100 mm und mehr bevorzugt zwischen 60 mm und 80 mm beträgt.

9. Saugfähiger Artikel (1) nach Anspruch 8, wobei der Höcker (8) weiter einen ersten Schnittpunkt (P1) zwischen dem ersten Längsseitenrand (9) des Höckers (8) und dem rückseitigen quer verlaufenden Seitenrand (12) des Höckers (8), einen zweiten Schnittpunkt (P2) zwischen dem zweiten Längsseitenrand (10) des Höckers (8) und dem rückseitigen quer verlaufenden Seitenrand (12) des Höckers (8), und eine imaginäre gerade Linie (L1), die sich in die Querrichtung (X) des Artikels (1) von dem ersten Schnittpunkt (P1) zu dem zweiten Schnittpunkt (P2)) erstreckt und einen imaginären Punkt (Z1), positioniert in der Mitte der imaginären geraden Linie (L1), umfasst, wobei der vordere quer verlaufende Seitenrand (11) des Höckers (8) weiter einen äußersten Punkt (Y2), positioniert in einer Längsrichtung (Y) am nächsten zum vorderen Abschnitt (2) des Artikels (1), umfasst, wobei der Abstand (A) der längste Abstand des Höckers (8) in einer Längsrichtung (Y) des Artikels zwischen dem äußersten Punkt (Y2) und dem imaginären Punkt (Z1) ist.

10. Saugfähiger Artikel (1) nach einem der vorstehenden Ansprüche, wobei der Höcker (8) eine Dicke (B) zwischen 0,5 mm und 30 mm, vorzugsweise zwischen 1 mm und 10 mm und mehr bevorzugt zwischen 2 mm und 5 mm aufweist.

11. Saugfähiger Artikel (1) nach einem der vorstehenden Ansprüche, wobei ein Abstand (C) zwischen dem ersten und zweiten Längsseitenrand (9, 10) des Höckers (8) zwischen 20 mm und 70 mm, vorzugsweise zwischen 30 mm und 40 mm beträgt.

12. Saugfähiger Artikel (1) nach Anspruch 9, wobei der Abstand (C) der längste Abstand in einer Querrichtung (X) des Höckers (8) zwischen dem ersten und zweiten Längsseitenrand (9, 10) des Höckers (8) ist.

13. Saugfähiger Artikel (1) nach einem der vorstehenden Ansprüche, wobei der rückseitige quer verlaufende Seitenrand (12) des Höckers (8) weiter einen innersten Punkt (Y1), positioniert in einer Längsrichtung (Y) am nächsten zum vorderen Abschnitt (2) des Artikels (1), umfasst, wobei ein Abstand D1 zwischen dem innersten Punkt (Y1) und dem imaginären Punkt (Z1) zwischen 5 mm und 50 mm und mehr bevorzugt zwischen 10 mm und 25 mm beträgt.

14. Saugfähiger Artikel (1) nach einem der vorstehenden Ansprüche, wobei der Höcker (8) weiter einen dritten Schnittpunkt (P3) zwischen dem ersten Längsseitenrand (9) des Höckers (8) und dem vorderen quer verlaufenden Seitenrand (11) des Höckers (8), einen vierten Schnittpunkt (P4) zwischen dem zweiten Längsseitenrand (10) des Höckers (8) und dem vorderen quer verlaufenden Seitenrand (11) des Höckers (8), umfasst, wobei sich eine imaginäre gerade Linie (L2) in die Querrichtung (X) des Artikels (1) von dem dritten Schnittpunkt (P3) zu dem vierten Schnittpunkt (P4) erstreckt, und ein imaginärer Punkt (Z2) in der Mitte der imaginären geraden Linie (L2) positioniert ist, wobei ein Abstand D2 zwischen dem äußersten Punkt (Y2) und dem imaginären Punkt (Z2) zwischen 5 mm und 50 mm und mehr bevorzugt zwischen 10 mm und 25 mm beträgt.

15. Saugfähiger Artikel (1) nach einem der Ansprüche 13 und 14, wobei der Abstand D1 mit dem Abstand D2 identisch ist.

16. Saugfähiger Artikel (1) nach einem der vorstehenden Ansprüche, wobei der Höcker (8) aus einem High-Loft-Vlies-Material, einem Airlaid-Material oder einem Schaumstoffmaterial besteht.

## Revendications

1. Article absorbant (1), l'article absorbant (1) étant un article d'hygiène personnelle, lequel article a une direction longitudinale (Y) et une direction transversale (X), une partie frontale (2), une partie arrière (4) et une partie d'entre-jambes (3) située entre la partie arrière (4) et la partie frontale (2), l'article comprenant une feuille supérieure perméable aux liquides (5), une feuille de fond imperméable aux liquides (6), et un noyau absorbant (7) enfermé entre la feuille supérieure (5) et la feuille de fond (6), et une bosse (8) disposée substantiellement dans la partie d'entre-jambes (3) entre la feuille supérieure (5) et la feuille de fond (6) et s'étendant dans la direction longitudinale (Y) de l'article
**caractérisé par**
ladite bosse (8) ayant une forme essentiellement rectangulaire avec un premier et un deuxième bord latéral longitudinal (9,10) s'étendant dans la direction longitudinale (Y) de l'article (1), un bord latéral transversal frontal (11) s'étendant dans la direction transversale (X) vers la partie frontale (2) de l'article (1), un bord latéral transversal arrière (12) s'étendant dans la direction transversale (X) vers la partie arrière (4) de l'article (1), dans lequel ledit bord latéral transversal frontal (11) de ladite bosse (8) comprend une protubérance (13), dans lequel la protubérance (13) comprend un contour s'étendant entre lesdits premier et deuxième bords latéraux longitudinaux (9, 10) de la bosse (8), ledit contour a une forme sinusoïdale, et ledit bord latéral transversal arrière (12) de ladite bosse (8) comprend un évidement (14), dans lequel ledit évidement (14) comprend un contour s'étendant entre lesdits premier et deuxième bords latéraux longitudinaux (9, 10) de la bosse (8), ledit contour a une forme sinusoïdale, dans lequel les contours respectifs sont essentiellement identiques,
ledit article (1) ayant un plan horizontal s'étendant dans lesdites directions longitudinale et transversale (9, 10) de l'article (1), dans lequel ladite protubérance (13) s'étend substantiellement dans ledit plan horizontal, et/ou dans lequel ledit évidement (14) s'étend substantiellement dans ledit plan horizontal.

2. Article absorbant (1) selon la revendication 1, dans lequel ladite bosse (8) est agencée entre la feuille supérieure (5) et le noyau absorbant (7).

3. Article absorbant (1) selon l'une quelconque des revendications précédentes, dans lequel ladite protubérance (13) a une forme convexe.

4. Article absorbant (1) selon l'une quelconque des revendications précédentes, dans lequel ledit évidement (14) a une forme concave.

5. Article absorbant (1) selon l'une quelconque des revendications précédentes, dans lequel ledit article absorbant (1) comprend en outre une couche supplémentaire (15) positionnée entre ladite feuille supérieure perméable aux liquides (5) et ladite bosse (8).

6. Article absorbant (1) selon l'une quelconque des revendications précédentes, dans lequel ledit article absorbant (1) comprend en outre une couche supplémentaire (15) positionnée entre ladite bosse (8) et ledit noyau absorbant (7).

7. Article absorbant (1) selon l'une quelconque des revendications 5 ou 6, dans lequel ladite couche supplémentaire (15) est une couche d'acquisition de liquide (15).

8. Article absorbant (1) selon l'une quelconque des revendications précédentes, dans lequel une distance (A) entre ledit bord latéral transversal frontal (11) de ladite bosse (8) et ledit bord latéral transversal arrière (12) de ladite bosse (8) est entre 20mm et 120mm, de préférence entre 40mm et 100mm et plus préférablement entre 60mm et 80mm.

9. Article absorbant (1) selon la revendication 8, dans laquelle ladite bosse (8) comprend en outre un premier point d'intersection (P1) entre ledit premier bord latéral longitudinal (9) de la bosse (8) et ledit bord latéral transversal arrière (12) de la bosse (8), un deuxième point d'intersection (P2) entre ledit deuxième bord latéral longitudinal (10) de la bosse (8) et ledit bord latéral transversal arrière (12) de la bosse (8), et une ligne droite imaginaire (L1) s'étendant dans la direction transversale (X) dudit article (1) dudit premier point d'intersection (P1) au dit deuxième point d'intersection (P2) et un point imaginaire (Z1) positionné au centre de ladite ligne droite imaginaire (L1), dans lequel ledit bord latéral transversal frontal (11) de la bosse (8) comprend en outre un point ultrapériphérique (Y2) positionné dans une direction longitudinale (Y) la plus proche de la partie frontale (2) de l'article (1), ladite distance (A) étant la distance la plus longue de la bosse (8) dans une direction longitudinale (Y) de l'article entre ledit point ultrapériphérique (Y2) et ledit point imaginaire (Z1).

10. Article absorbant (1) selon l'une quelconque des revendications précédentes, dans lequel ladite bosse (8) a une épaisseur (B) comprise entre 0,5mm et 30mm, préférablement entre 1mm et 10mm et plus préférablement entre 2mm et 5mm.

11. Article absorbent (1) selon l'une quelconque des revendications précédentes dans lequel une distance (C) entre lesdits premier et deuxième bords latéraux longitudinaux (9, 10) de ladite bosse (8) est comprise entre 20mm et 70mm, préférablement entre 30mm et 40mm.

12. Article absorbant (1) selon la revendication 9, dans laquelle ladite distance (C) est la distance la plus longue dans une direction transversale (X) de la bosse (8) entre lesdits premier et deuxième bords latéraux longitudinaux (9, 10) de ladite bosse (8).

13. Article absorbant (1) selon l'une quelconque des revendications précédentes, dans lequel ledit bord latéral transversal arrière (12) de la bosse (8) comprend en outre un point ultrapériphérique (Y1) positionné dans une direction longitudinale (Y) la plus proche de la partie frontale (2) de l'article (1), dans laquelle une distance D1 entre ledit point ultrapériphérique (Y1) et ledit point imaginaire (Z1) est comprise entre 5mm et 50mm et plus préférablement entre 10mm et 25mm.

14. Article absorbant (1) selon l'une quelconque des revendications précédentes, dans lequel ladite bosse (8) comprend en outre (P3) entre ledit premier bord latéral longitudinal (9) de la bosse (8) et ledit bord latéral transversal frontal (11) de la bosse (8), un quatrième point d'intersection (P4) entre ledit deuxième bord latéral longitudinal (10) de la bosse (8) et ledit bord latéral transversal frontal (11) de la bosse (8), une ligne droite imaginaire (L2) s'étendant dans la direction transversale (X) dudit article (1) à partir dudit troisième point d'intersection (P3) vers ledit quatrième point d'intersection (P4), et une ligne imaginaire (Z2) positionnée au centre de ladite droite imaginaire (L2), dans lequel une distance D2 entre ledit point ultrapériphérique (Y2) et ledit point imaginaire (Z2) est comprise entre 5mm et 50mm et plus préférablement entre 10mm et 25mm.

15. Article absorbant (1) selon l'une quelconque des revendications 13 et 14, dans lequel ladite distance D1 est identique à ladite distance D2.

16. Article absorbant (1) selon l'une quelconque des revendications précédentes, dans lequel ladite bosse (8) est constituée d'un matériau matériel à haut volume non-tissé, d'un matériau appliqué par jet d'air (airlaid) ou d'un matériau mousseux.
